# EUROPEAN PATENT APPLICATION

(11) **EP 0 609 794 A1**
(43) Date of publication of application: **10.08.1994**
(21) Application number: 94101339.3
(22) Date of filing: 29.01.1994
(51) Int. Cl.: A61L 33/00

(54) **A blood collection device**

(30) Priority: 01.02.1993 JP 14765/93
(71) Applicant: Becton Dickinson and Company, Franklin Lakes, New Jersey 07417-1880 (US)
(72) Inventor: Reichenbach, Judith A., Pompton Plains, New Jersey 07444 (US)
(74) Representative: von Kreisler, Alek, Dipl.-Chem.

(57) **Abstract**

A blood collection device for chemical analysis of plasma that is spray coated with an anticoagulant.

## Description

### Field of the invention

The present invention relates to a blood collection device used for chemical analysis of plasma. More specifically, the present invention pertains to more precise anticoagulant fill and improved dissolution into the blood specimen.

### Prior Art

As more and more medical appliances made of plastic materials are used frequently and diversly, there has been a demand for improved anticoagulant fill into the appliances. Such improvement is conducive to collection tubes, especially blood collection tubes among plastic medical appliances. Blood collection tubes need to be designed in such a manner that anticoagulant filled in tubes efficiently works in tests or analysis, and the materials of the tubes do not interfere with testing or analysis. Such tests include but are not limited to hematology, blood chemistry, blood typing, toxicology analysis and therapeutic drug monitoring.

Freeze drying, vacuum drying, liquid filling and residual wall coating can be used as conventional methods for filling anticoagulant into blood collection devices. However, these conventional methods have the following disadvantages. In the case of freeze-drying, anticoagulant can be rehydrated again after drying, which is not desirable. With drying methods, the anticoagulant tens to be localized within the tube. In addition, liquid filling is not practical for heparin tubes as the liquid anticoagulant will degrade with irradiation during sterilization.

Furthermore, these conventional methods do not employ water exclusively as a solvent. For example, in a residual wall coating process, a carrier/binding agent such as silicone is required. Additives add expense to the product and increase the probability of interaction with other product components and the blood specimen which may alter results obtained from the collection of the blood specimen. All methods may require a volatile solvent such as freon in order to facilitate drying at low temperatures which will not damage the plastic tube.

### Problems to be solved by the invention

The object of this invention is to solve the aforementioned problems: In other words, the object of this invention is more precise, uniform and stable dry anticoagulant fill and improved dry anticoagulant dissolution into the blood specimen. Another object of the invention is to attain this improvement without employing solvents which may be detrimental to the environment.

### Means for solving the problems

The present invention relates to a blood collection device for chemical analysis of plasma specimen.

Said device is a test tube made of polyethylene terephthalate, etc., onto an interior wall of which anticoagulant, such as lithium heparin or sodium heparin, is applied by a spray coating process.

Anticoagulant is applied onto an interior wall in the form of fine mist by a spray coating process using a carrier solvent, preferably water. The main advantages of the plastic tube with spray coated anticoagulant on the tube interior wall are more precise, stable, uniform and dry anticoagulant fill and improved dry anticoagulant dissolution into blood specimen. Because of the fine mist of anticoagulant, the actual surface area of anticoagulant exposed to blood is maximized.

According the present invention, the precision of the anticoagulant fill is improved by tailoring the solution concentration and dispensed volume. That is, a high precision metering system can be used to deliver the solution to the spray coating system. This is the only method for precise and efficient use of heparin in a uniformly distributed manner. This method improves the delivery of the anticoagulant solution to a plastic tube, rectifies unprecise distribution of anticoagulant onto the wall of the tube and facilitates dissolution of anticoagulant, upon blood collection.

It is preferable that the anticoagulant solution is metered and dispensed by a volumetric type device, such as a positive displacement pump. The solution concentration (amount of anticoagulant per unit volume of solution) is tailored with the dispense volume so that the desired amount of anticoagulant is dispensed into the device.

A blood collection device of the present invention can be either an evacuated blood collection device of a non-evacuated blood collection device.

According to the present invention, anticoagulant dissolution is improved by a number of factors. First, the plastic material used for the tube has a lower surface energy than that of glass. The lower surface energy results in the anticoagulant remaining on the tube wall after the spray coat application rather than running down the tube wall and gathering in the bottom of the tube. With the anticoagulant remaining where applied inside the tube, the anticoagulant can be applied along the entire internal surface area of the tube. This makes the anticoagulant available to more of the specimen and decreases the anticoagulant dissolution time into the specimen. Also, by making the anticoagulant available to more of the specimen, specimen microclots is decreased.

In addition, according to the blood collection device and the method for making thereof of the present invention, water can be used as the solvent without other volatile solvents such as freon. Water has an advantage in that it has minimal detrimental effects on product or environment.

Furthermore, there is a cost advantage with the device and the method for making the same according to the present invention. The increased precision in an anticoagulant fill enables lower amounts of heparin to be used. As heparin is expensive, cost reductions can be realized.

It is obvious for those having ordinary skill in the art that many modifications can be made to the present invention without departing from the scope and spirit thereof.

The present invention will be further explained by way of non-limiting examples.

### Example

13,931 mg of lithium heparin dissolved in 250 ml of sterilized distilled water was prepared. The coating solution was then sprayed in a fine mist onto the interior walls of 10,000 pieces of polyethylene terephthalate test tubes.

The coating solution on the test tubes was then dried by forced hot air. Thus, test tubes with a dispensed coating of lithium heparin on the interior walls were obtained. The test tubes were then each evacuated and sealed with a closure and sterilized by gamma irradiation at 2.5 mega Rads.

## Claims

1. A plastic blood collection device comprising a top end, a bottom end, a sidewall extending from said top end to said bottom end and including an exterior and interior surface, and a spray coated anticoagulant on said interior surface applied by a spray coating process which employs water as a carrier solvent which is subsequently substantially removed leaving a dry additive.

2. The device of Claim 1, wherein said plastic is polyethylene terephthalate.

3. The device of Claim 1 or 2, wherein said blood collection device is a tube.

4. The device of any one of Claims 1 to 3, wherein the said anticoagulant is heparin.

5. The device of Claim 4, wherein the concentration of heparin is about 1,000 units per ml.

6. A method for coating substrates characterized by the steps of:
(a) preparing a coating solution of anticoagulant and water;
(b) selecting a substrate to be coated;
(c) dispersing said coating solution to said substrate in a fine mist; and
(d) drying said applied coating by applying forced air to the coated substrate for a sufficient period of time to dry the coating whereby a dry additive remains.

7. The method of Claim 6, wherein said substrate is a plastic material.

8. The method of Claim 6 or 7, wherein the said substrate is a blood collection device.

9. The method of any of Claims 6 to 8, wherein the said anticoagulant is heparin.

10. The method of Claim 9, wherein the concentration of heparin is about 1,000 units per ml.

11. The device of any Claims 1 to 5, wherein said spray coating process comprises:
(a) preparing a coating solution of anticoagulant and water;
(b) dispersing said coating solution to said tube in a fine mist; and
(c) drying said applied coating by applying forced air to the coated tube for sufficient period of time to dry the coating whereby a dry additive remains.
